# EUROPEAN PATENT SPECIFICATION

(11) **EP 2 219 665 B1**
(45) Date of publication and mention of the grant of the patent: **16.03.2011**
(21) Application number: 08842081.5
(22) Date of filing: 27.10.2008
(51) Int. Cl.: A61K 38/29, A61K 47/26, A61K 47/32, A61K 47/18, A61K 9/19

(54) **PARATHYROID HORMONE FORMULATIONS AND USES THEREOF**
PARATHORMON-FORMULIERUNGEN UND IHRE ANWENDUNGEN
FORMULATIONS DE L'HORMONE PARATHYROÏDIENNE ET UTILISATIONS DE CELLES-CI

(30) Priority: 26.10.2007 EP 07021017; 26.10.2007 US 996061 P
(43) Date of publication of application: 25.08.2010
(73) Proprietor: Nycomed Danmark ApS, 4000 Roskilde (DK)
(72) Inventor: LOER LINDEROTH, Dorte, DK-3450 Allerod (DK); LINDEGAARD HANSEN, Lars, DK-4000 Roskilde (DK)
(74) Representative: Wichmann, Hendrik
(86) International application number: PCT/EP2008/009069
(87) International publication number: WO 2009/053106

(56) References cited:
- WO-A-2005/051456
- WO-A-2007/044375
- US-A1- 2007 134 279
- US-B1- 6 416 503

## Description

### BACKGROUND

Parathyroid hormone (PTH) is a secreted, 84 amino acid product of the mammalian parathyroid gland that controls serum calcium levels through its action on various tissues, including bone. Studies in humans with certain forms of PTH have demonstrated an anabolic effect on bone, and have prompted significant interest in its use for the treatment of osteoporosis and related bone disorders.

Human PTH may be obtained through tissue extraction, from peptide synthesis or from genetically engineered yeast, bacterial or mammalian cell hosts. Essentially pure human PTH is disclosed in U.S. Pat. No. 5,208,041. Recombinant production of PTH in E. coli is disclosed, for example, in U.S. Pat. No. 5,223,407, U.S. Pat. No. 5,646,015 and U.S. Pat. No. 5,629,205. Production of recombinant human PTH in yeast is disclosed in EP-B-0383751. Synthetic human PTH is commercially available from Bachem Inc., Bubendorf, Switzerland. In addition, formulations of PTH are described in, for example, U.S. Patent 5,496,801 and U.S. Published App. No. 2005/0209144 A1.

Despite its usefulness, achieving commercially viable formulations of parathyroid hormone that are suitable for delivery to a subject in need has proven challenging. Because it is a protein and far more labile than the traditional small molecule drugs, PTH is susceptible to both chemical and physical degradation. Moreover, PTH is sensitive to oxidation. For example, the PTH molecule is sensitive to oxidation at the Met₈ and Met₁₈ residues. Yet, in order to preserve the bioactivity of PTH, it is believed that the N-terminal sequence of the molecule must remain intact. Additionally, when formulated in solution, PTH exhibits a tendency to form aggregates as the concentration of PTH increases.

Though commercially viable formulations of PTH have been achieved, such formulations typically require particular handling or storage conditions, such as refrigeration, in order to maintain the physical and chemical stability of the PTH to be delivered. Such handling requirements can prove inconvenient and, possibly, decrease patient compliance. In addition, even when the particular handling requirements for a given formulation are followed, it is often the case that solution formulations of PTH exhibit a useful life of days to weeks.

WO 2005/027978 discloses novel derivatives of glucagone-like-peptide-1 (GLP-1) and methods of making and using them.
EP 1 598 074 A1 discloses stabilized preparations containing protein.
WO 2004/007520 describes methods and compositions for preventing oxidative degradation of proteins.
WO 2005/028516 A2 relates to a drug delivery system, based on novel compounds with high affinity to plasma proteins.
WO 2006/017852 discloses pharmaceutical compositions for controlled release delivery of biologically active compounds.

### SUMMARY OF THE INVENTION

Formulations of PTH that may be administered to animal or human subjects are provided herein. In one embodiment, the PTH formulations described herein is in the form of a stable solution formulation comprising PTH, methionine, at least one Poloxamer surfactant, and a physiologically acceptable buffering agent. Also described herein are methods of making and using the PTH formulations described herein.

### DESCRIPTION OF THE INVENTION

PTH formulations that may be administered to human or animal subjects are described herein. In one embodiment, the PTH formulations described herein are solution formulations that include PTH and at least one antioxidant. Alternatively, the PTH formulations described herein are solution formulations that include at least one non-ionic surfactant and, optionally, at least one antioxidant. In one embodiment, a PTH formulation as described herein is a stable solution formulation that includes PTH and at least one antioxidant. In another embodiment, a PTH formulation as described herein is a stable solution formulation that includes at least one non-ionic surfactant and, optionally, at least one antioxidant. The PTH formulations described herein may also include one or more additional constituents, such as, for example, one or more of a buffer, tonicity modifier or preservative. Methods of making and using the PTH formulation provided herein are also described.

As it is used herein, the term "stable" preferably refers to solution formulations wherein the purity of the PTH included in the formulation is greater than 90%, as measured by reverse phase high performance liquid chromatography (RP-HPLC) and cation exchange high performance liquid chromatography (CE-HPLC), after storage of the formulation at 25° C for 30 days. In one embodiment, the purity of the PTH included in a PTH formulation as described herein is 92% or greater after storage of the formulation at 25° C for 30 days. In another embodiment, the purity of the PTH included in a PTH formulation as described herein is 95% or greater after storage of the formulation at 25° C for 30 days. Alternatively, the term "stable" refers to solution formulations wherein the purity of the PTH included in the formulation is 80% or greater, as measured by RP-HPLC and CE-HPLC, after storage of the formulation at 25° C for 60 days. In one embodiment, the purity of the PTH included in a PTH formulation as described herein is greater than 80% after storage of the formulation at 25° C for 60 days. In another embodiment, the purity of the PTH included in a PTH formulation as described herein is 85% or greater after storage of the formulation at 25° C for 60 days.

In another aspect, the solution formulations according to the present description have been shown to exhibit improved clarity, visual appearance and less precipitation under stressed conditions like e.g. shaking. These improvements can e.g. be shown as measured by turbidimetry or UV-VIS spectroscopy.

The PTH formulations described herein may be prepared as a lyophilized formulation that is reconstituted prior to administration to a subject in need thereof. Alternatively, the PTH formulations described herein may be prepared as a ready to administer solution. Whether the formulation is reconstituted or prepared as a solution, the formulation will include a suitable vehicle. For example, the vehicle may be a water-based vehicle, such as sterile water or saline.

The PTH formulations described herein incorporate PTH in a medically effective amount, a term used with reference to amounts useful either therapeutically or in medical diagnosis. The particular amount of parathyroid hormone incorporated in the preparation can be determined based on the type of PTH selected and the intended end-use of the preparation. In one embodiment, the PTH consists of a peptide or a modified peptide, such as a peptide conjugate. In one embodiment, the present PTH formulation may include the full-length, 84 amino acid form of human PTH, such as obtained recombinantly, by peptide synthesis or by extraction. Where the human form of the full-length PTH hormone is referred to herein, the abbreviation "hPTH(1-84)" is used. For purposes of this disclosure, hPTH(1-84) has the amino acid sequence reported by Kimura et al., Biochem Biophys Res Comm, 114 (2):493.

In another embodiment, the PTH included in the PTH formulation as described herein is a homolog or variant of hPTH(1-84) that is biologically active. The term "biologically active" refers to PTH molecules that are active in a bioassay for PTH activity, such as the rat osteosarcoma cell-based assay for PTH-stimulated adenylate cyclase production (See, Rodan et al. (1983) J. Clin. Invest. 72,1511; and Rabbani et al. (1988) Encrinol. 123, 2709). Said homolog can for example be a peptide with at least 80% sequence identity, such as at least 85% sequence identity, for example at least 90% sequence identity, such as at least 95% sequence identity, for example at least 98% sequence identity, with hPTH(1-84). The terms " homolog" or "variant" can include, for example, truncated PTH molecules such as PTH(1-34).

In yet another embodiment, a PTH formulation as described herein may include the full-length form of a PTH molecule occurring in a non-human mammal. In one such embodiment, the PTH molecule may be a bovine or porcine form of PTH as described by Keutmann et al., Current Research on Calcium Regulating Hormones, Cooper, C.W. (ed.), 1987, University of Texas Press, Austin, pp 57-63. In another embodiment, a PTH preparation as described herein may be prepared using a homolog or variant of a non-human, mammalian PTH molecule that is biologically active.

In yet another embodiment, a PTH formulation as described herein may include a variant of hPTH(1-84) or a variant of a non-human, mammalian PTH molecule that includes from 1 to 5 amino acid substitutions. Such substitutions can be effected to alter the stability of the molecule upon formulation or the half-life of the molecule upon administration. With respect to hPTH(1-84), such amino acid substitutions may include the replacement of the methionine residues at positions 8 or 18 with a leucine residue or another hydrophobic amino acid that improves the stability of the molecule against oxidation. Another amino acid substitution may be the replacement of amino acids at positions 25-27 with trypsin insensitive amino acids, such as histidine, or another amino acid that improves the stability of the molecule against proteases.

In one embodiment, the PTH included in a formulation as described herein may be provided at a concentration of from 0.1 mg/ml to 10 mg/ml, such as, for example, 0.3 mg/ml to 10 mg/ml, 0.3 mg/ml to 5 mg/ml, 0.3 mg/ml to 3 mg/ml, 0.3 mg/ml to 2 mg/ml, or 0.3 mg/ml to 1 mg/ml, such as, for example, from 0.4 mg/ml to 8 mg/ml. In another embodiment, the PTH included in a formulation as described herein may be provided at a concentration ranging above 1 mg/ml, such as, for example, 1 mg/ml to 10 mg/ml, 1 mg/ml to 5 mg/ml, 1 mg/ml to 3 mg/ml, or 1 mg/ml to 2 mg/ml. In a further embodiment, the PTH is included at a concentration selected from 1 mg/ml to 5 mg/ml, 1mg/ml to 3 mg/ml and 1 mg/ml to 2 mg/ml. In one embodiment, the PTH may be hPTH(1-84) provided at a concentration of 0.3 mg/ml to 3 mg/ml. In another embodiment, the PTH may be hPTH(1-84) provided at a concentration of 1 mg/ml to 3 mg/ml. In yet another embodiment, the PTH may be hPTH(1-84) provided at a concentration of 1 mg/ml to 2 mg/ml, such as about 1.4 mg/ml. If the PTH used is an analogue, variant or homologue of hPTH(1-84), it is understood by one skilled in the art that PTH concentrations giving a functionally equivalent effect to hPTH(1-84) at the above-mentioned concentrations can also be employed.

In one embodiment, a PTH formulation as described herein includes at least one antioxidant. Examples of antioxidants that may be used in a PTH formulation according to the present description include butylhydroxytoluen (BHT), butylhydroxyanisol (BHA), tocopherol (Vitamin E), propylgallate, ascorbic acid, sodium bisulfite, monothioglycerol, methionine, and EDTA (sodium, dicalcium, anhydrous). Where desired, a PTH formulation according to the present description may include two or more antioxidants. In one such embodiment, the PTH formulation includes a combination of two or more of the antioxidants described herein. In one embodiment of a PTH formulation including at least one antioxidant, one or more antioxidants are provided at a concentration of up to about 30 mg/ml. For example, one or more antioxidants may each be provided at a concentration of 1 mg/ml to 10 mg/ml, 2 mg/ml to 9 mg/ml, 3 mg/ml to 8 mg/ml, 4 mg/ml to 7 mg/ml, or 5 mg/ml to 6 mg/ml. Alternatively, one or more antioxidants may each be provided at a concentration of 10 mg/ml to 20 mg/ml, 11 mg/ml to 19 mg/ml, 12 mg/ml to 18 mg/ml, 13 mg/ml to 17 mg/ml, or 14 mg/ml to 16 mg/ml. Even further, one or more antioxidants may each be provided at a concentration of 1 mg/ml to 5 mg/ml, 1 mg/ml to 4 mg/ml, 1 mg/ml to 3 mg/ml, or 1 mg/ml to 2 mg/ml. In one embodiment, methionine may be provided as an antioxidant at a concentration of up to 20 mg/ml. For example, a PTH formulation as described herein may include methionine as an antioxidant at a concentration selected from up to 5 mg/ml, 2 mg/ml to 16 mg/ml, 15 mg/ml to 20 mg/ml, 1 mg/ml to 4 mg/ml,15 mg/ml to 17 mg/ml, and 2 mg/ml to 4 mg/ml, such as about 2.0 mg/ml. In another embodiment of a PTH formulation according to the present description, EDTA (sodium, dicalcium, anhydrous) is provided as an antioxidant, which may, for example, be provided at a concentration of up to 1 mg/ml.

As defined herein, methionine is intended to encompass either D-methionine, or L-methionine, or a mixture thereof, preferably L-methionine.

In another embodiment, a PTH formulation according to the present description includes at least one pharmaceutically acceptable, non-ionic surfactant. Non-ionic surfactants that may be included in a PTH formulation as described herein include, for example, α-hydro-ω-hydroxypoly(oxyethylene)poly(oxypropylene)poly(oxyethylene) block copolymer (e.g., a Poloxamer surfactant), polyoxyethylene sorbitan fatty acid esters (e.g., a Tween surfactant), polyoxyethylene alkyl ethers (e.g., a Brij surfacant), polyethylene glycol tert-octylphenyl ether (e.g., a Triton-X surfactant), and polyvinylalcohol (PVA) surfactants. Where desired, a PTH formulation as described herein may include two or more non-ionic surfactants. In one such embodiment, the PTH formulation includes two or more of the non-ionic surfactants described herein, with each of the non-ionic surfactants being provided at a concentration according to the teachings set forth below. Each of the non-ionic surfactants described herein is commercially available in one or more different grades.

In one embodiment, the at least one non-ionic surfactant includes a Poloxamer surfactant. In one such embodiment, the at least one non-ionic surfactant includes a Poloxamer surfactant at a concentration of up to 2 mg/ml. For example, a Poloxamer surfactant may be included at a concentration of 0.02 mg/ml to 2 mg/ml, 0.05 mg/ml to 0.5 mg/ml, 0.05 mg/ml to 0.75 mg/ml, 0.05 mg/ml to 0.3 mg/ ml, 0.2 mg/ml to 0.5 mg/ml, 0.3 mg/ml to 0.5 mg/ml, or 0.3 mg/ml to 1 mg/ml, such as about 0.3 mg/ml. In one embodiment, the at least one Poloxamer surfactant is provided at a concentration from 0.02 mg/ml to 1 mg/ml, preferably at a concentration selected from 0.05 mg/ml to 0.5 mg/ml, 0.05 mg/ml to 0.75 mg/ml, 0.05 mg/ml to 0.3 mg/ml, 0.2 mg/ml to 0.5 mg/ml, 0.3 mg/ml to 0.5 mg/ml, and 0.3 mg/ml to 1 mg/ml. In one embodiment, the Poloxamer surfactant may be Poloxamer 188 provided at a concentration selected from, for example, 0.05 mg/ml to 0.3 mg/ml, 0.05 mg/ml to 0.5 mg/ml, or 0.3 mg/ml to 1 mg/ml. In another embodiment, the Poloxamer surfactant may be Poloxamer 237 provided at a concentration selected from, for example, 0.05 mg/ml to 0.3 mg/ml, 0.05 mg/ml to 0.5 mg/ml, or 0.3 mg/ml to 1 mg/ml. In another embodiment, the Poloxamer surfactant may be Poloxamer 338 provided at a concentration selected from, for example, 0.05 mg/ml to 0.3 mg/ml, 0.05 mg/ml to 0.5 mg/ml, or 0.3 mg/ml to 1 mg/ml. In another embodiment, the Poloxamer surfactant may be Poloxamer 407 provided at a concentration selected from, for example, 0.05 mg/ml to 0.3 mg/ml, 0.05 mg/ml to 0.5 mg/ml, or concentration of 0.3 mg/ml to 1 mg/ml. In yet another embodiment, the Poloxamer surfactant may be Poloxamer 124 provided at a concentration selected from, for example, 0.05 mg/ml to 0.3 mg/ml, 0.05 mg/ml to 0.5 mg/ml, or 0.3 mg/ml to 1 mg/ml. In yet another embodiment, a PTH formulation according to the present description may include two or more Poloxamer surfactants. Where a PTH formulation includes two or more Poloxamer surfactants, each Poloxamer surfactant may, for example, be provided at one of the preceding concentrations. Poloxamers are well-known to the skilled person (see, e.g., Fiedler, Lexikon der Hilfsstoffe, 5th Ed.; Vol. 2, page 1334).

In another embodiment, the at least one non-ionic surfactant includes a Tween surfactant. Suitable Tween surfactants include, for example, Tween 20, Tween 40 and Tween 80. In one such embodiment, the PTH formulation includes a Tween 20 surfactant included, for example, at a concentration of 0.02 mg/ml to 2 mg/ml, such as 0.02 mg/ml to 0.1 mg/ml. In another such embodiment, the PTH formulation includes a Tween 40 surfactant included, for example, at a concentration of 0.02 mg/ml to 2 mg/ml, such as 0.02 mg/ml to 0.1 mg/ml. In yet another such embodiment, the PTH formulation includes a Tween 80 surfactant included, for example, at a concentration of 0.02 mg/ml to 2 mg/ml, such as 0.02 mg/ml to 0.1 mg/ml. In yet another embodiment, a PTH formulation according to the present description may include two or more Tween surfactants. Where a PTH formulation includes two or more Tween surfactants, each Tween surfactant can for example be provided at one of the preceding concentrations.

In another embodiment, the at least one non-ionic surfactant may be a Brij surfactant. Suitable Brij surfactants include, for example, Brij 35 and Brij 52. In one such embodiment, the PTH formulation includes a Brij-52 surfactant, and the Brij-52 surfactant may be included, for example, at a concentration of 0.05mg/ml to 2 mg/ml or 0.02 mg/ml to 0.1 mg/ml. In another such embodiment, the PTH formulation includes a Brij-35 surfactant, and the Brij-35 surfactant may be included, for example, at a concentration of 0.02 mg/ml to 2 mg/ml, such as 0.02 mg/ml to 0.5 mg/ml. In yet another embodiment, a PTH formulation according to the present description may include two or more Brij surfactants. Where a PTH formulation includes two or more Brij surfactants, each Brij surfactant may, for example, be provided at one of the preceding concentrations.

In another embodiment, the at least one non-ionic surfactant includes a Triton-X surfactant, such as, for example, Triton X-100. Where a PTH formulation includes a Triton-X surfactant, the Triton-X surfactant can, for example, be included at a concentration of 0.05 mg/ml to 2 mg/ml, such as 0.1 mg/ml to 0.3 mg/ml. In one such embodiment, two or more Triton-X surfactants may be included in a PTH formulation as described herein, for example with each of the Triton-X surfactants provided at one of the preceding concentrations.

In another embodiment, the at least one non-ionic surfactant includes a PVA surfactant, such as, for example, PVA 4-88. Where a PTH formulation includes a PVA surfactant, the surfactant can, for example, be included at a concentration of up to 15 mg/ml. For example, a PVA surfactant may be included at a concentration of 1 mg/ml to 15 mg/ml, 1 mg/ml to 10 mg/ml, 1 mg/ml to 7 mg/ml, 1 mg/ml to 5 mg/ml, 1 mg/ml to 3 mg/ml, 1 mg/ml to 2 mg/ml, 2 mg/ml to 3 mg/ml, or 2 mg/ml to 10 mg/ml. Alternatively, a PVA surfactant may be included at a concentration of 5 mg/ml to 15 mg/ml, 5 mg/ml to 10 mg/ml, 5 mg/ml to 7 mg/ml, 7mg/ml to 10 mg/ml, or 9 mg/ml to 10 mg/ml. In one embodiment, the PVA surfactant may be PVA 4-88, provided, for example, at a concentration of 1 mg/ml to 15 mg/ml, 2mg/ml to 10 mg/ml or 1 mg/ml to 2 mg/ml. In another embodiment, the PVA surfactant may be PVA 4-88, provided, for example, at a concentration of 5 mg/ml to 7 mg/ml. In still another embodiment, the PVA surfactant may be PVA 4-88 provided at a concentration of 5 mg/ml to 10 mg/ml. In yet another embodiment, a PTH formulation as described herein may include two or more PVA surfactants, with each of the PVA surfactants provided at one of the preceding concentrations.

Thus, in one embodiment of a PTH formulation including at least one non-ionic surfactant as described herein, the at least one non-ionic surfactant is selected from Poloxamer 407, Poloxamer 338, Poloxamer 237, Poloxamer 188, Poloxamer 124, Tween 20, Tween 40, Tween 80, Brij 35, Brij 52, Triton X-100, PVA 4-88, and combinations of two or more of the forgoing. In particular, the above Poloxamers are well-known to the skilled person (see, e.g., Fiedler, Lexikon der Hilfsstoffe, 51h Ed.; Vol. 2, page 1334). In another embodiment of a PTH formulation including at least one non-ionic surfactant as described herein, the at least one non-ionic surfactant is selected from PVA 4-88, Brij 35, Brij 52, Triton X-100, and combinations of two or more of the forgoing.

Where a PTH formulation as described herein includes at least one non-ionic surfactant, it may additionally include at least one antioxidant. PTH formulations including at least one non-ionic surfactant as described herein may additionally include, for example, at least one antioxidant selected from butylhydroxytoluen (BHT), butylhydroxyanisol (BHA), tocopherol (Vitamin E), propylgallate, ascorbic acid, sodium bisulfite, monothioglycerol, methionine and EDTA (sodium, dicalcium, anhydrous). Where desired, a PTH formulation that includes at least one non-ionic surfactant may also include two or more antioxidants. In one such embodiment, the PTH formulation includes a combination of two or more of the antioxidants described herein. In one embodiment of a PTH formulation that includes at least one non-ionic surfactant and at least one antioxidant, one or more antioxidants may each be provided at a concentration of up to about 30 mg/ml. For example, in a PTH formulation that includes at least one non-ionic surfactant and at least one antioxidant, one or more antioxidants may each be provided at a concentration of 1 mg/ml to 10 mg/ml, 2 mg/ml to 9 mg/ml, 3 mg/ml to 8 mg/ml, 4 mg/ml to 7 mg/ml, or 5 mg/ml to 6 mg/ml. Alternatively, where a PTH formulation includes at least one non-ionic surfactant and at least one antioxidant, one or more antioxidants may each be provided at a concentration of 10 mg/ml to 20 mg/ml, 11 mg/ml to 19 mg/ml, 12 mg/ml to 18 mg/ml, 13 mg/ml to 17 mg/ml, or 14 mg/ml to 16 mg/ml. Even further, in a PTH formulation that includes at least one non-ionic surfactant and at least one antioxidant, one or more antioxidants may each be provided at a concentration of 1 mg/ml to 5 mg/ml, 1 mg/ml to 4 mg/ml, 1 mg/ml to 3 mg/ml, or 1 mg/ml to 2 mg/ml. In one embodiment of a PTH formulation that includes at least one non-ionic surfactant and at least one antioxidant, methionine may be provided as an antioxidant, and the methionine may be included at a concentration of up to 20 mg/ml. For example, a PTH formulation as described herein may include at least one non-ionic surfactant and methionine as an antioxidant, where the methionine is provided at a concentration selected from up to 5 mg/ml, 2 mg/ml to 16 mg/ml, 15 mg/ml to 20 mg/ml, 1 mg/ml to 4 mg/ml, 15 mg/ml to 17 mg/ml, and 2 mg/ml to 4 mg/ml. In one preferred embodiment, the antioxidant is methionine provided at a concentration of 1 mg/ml to 10 mg/ml, preferably 1 mg/ml to 5 mg/ml, 1 mg/ml to 4 mg/ml, 1 mg/ml to 3 mg/ml, 1 mg/ml to 2 mg/ml, 2 mg/ml to 9 mg/ml, 2 mg/ml to 4 mg/ml, 3 mg/ml to 8 mg/ml, 4 mg/ml to 7 mg/ml, or 5 mg/ml to 6 mg/ml. For example, a PTH formulation may comprise 0.5-5 mg/ml methionine and 0.1-0.6 mg/ml surfactant, such as poloxamer, for example poloxamer 188. Thus, one preferred example of a PTH formulation comprises about 2 mg/ml methionine and 0.3 mg/ml poloxamer 188 and optionally comprises one or more of the following: sodium chloride, citric acid monohydrate and mannitol. Another embodiment of a PTH formulation includes at least one non-ionic surfactant and at least one antioxidant, the antioxidant used may be EDTA (sodium, dicalcium, anhydrous), which may, for example, be provided at a concentration of up to 1 mg/ml.

Buffering agents that are pharmaceutically acceptable and suitable for maintaining the formulation at a physiologically acceptable pH may be use in a PTH formulation as described herein. The term "physiologically acceptable" indicates a pH of about 3.5 to about 7.5. In one embodiment, a buffer that maintains the pH of the formulation at a pH of 5 to 6.5 may be provided. In another embodiment, a non-volatile buffering agent may be provided. A "non-volatile buffering agent" is one that is not volatilized during a freeze-drying process to the extent that pH is reduced by more than 0.4 pH units. In yet another embodiment, a non-volatile buffering agent that maintains the pH of the formulation at a pH of 5 to 6.5 may be provided. In still another embodiment, a PTH formulation as described herein may be provided with a non-volatile buffering agent that maintains the pH of the composition at a pH of about 5.5. In yet another embodiment, two or more buffer systems may be utilized in order to maintain the pH of the PTH formulation at a desired level.

Pharmaceutically acceptable buffers that may be used in the PTH formulations described herein include, for example, acetate, citrate, phosphate, TRIS (Tromethamine), arginine and carbonate buffers. In one embodiment, citrate buffer is used. Where a citrate buffer is used as the buffering agent, it may, for example, be provided at a concentration of 5mM to 30 mM, 5 mM to 10 mM, or at a concentration of 10 mM. In one such embodiment, a PTH formulation as described herein is prepared with a citrate buffer at a concentration of 10 mM, and the citrate buffer is provided in a manner that maintains the pH of the formulation at a pH of 5.5. Other buffers are also suitable, for example at similar concentration and/or pH ranges as described above for the citrate buffer.

Thus, a pharmaceutical composition is disclosed comprising, when dissolved in a suitable vehicle, PTH at a concentration of 1 mg/ml to 10 mg/ml, methionine provided ata concentration of 1 mg/ml to 10 mg/ml, at least one Poloxamer surfactant provided at a concentration from 0.02 mg/ml to 1 mg/ml, and a physiologically acceptable buffering agent that maintains the pH of the formulation at a pH of 5 to 6.5.

In a preferred embodiment, the composition is in the form of a stable solution formulation.

In another preferred embodiment, the pharmaceutical composition comprises PTH at a concentration of 1.4 mg/ml, methionine at a concentration of 2 mg/ml, poloxamer 407 at a concentration of 0.3 mg/ml, and a physiologically acceptable buffering agent that maintains the pH of the formulation at a pH of 5.5.

In another preferrred embodiment, the pharmaceutical composition comprises PTH at a concentration of 1.4 mg/ml, methionine at a concentration of 2 mg/ml, poloxamer 188 at a concentration of 0.3 mg/ml, and a physiologically acceptable buffering agent that maintains the pH of the formulation at a pH of 5.5.

In still another preferred embodiment, the pharmaceutical composition comprises PTH at a concentration of 1.4 mg/ml, methionine at a concentration of 2 mg/ml, poloxamer 338 at a concentration of 0.3 mg/ml, and a physiologically acceptable buffering agent that maintains the pH of the formulation at a pH of 5.5.

In still another preferred embodiment, the pharmaceutical composition comprises PTH at a concentration of 1.4 mg/ml, methionine at a concentration of 2 mg/ml, poloxamer 338 at a concentration of 1.0 mg/ml, and a physiologically acceptable buffering agent that maintains the pH of the formulation at a pH of 5.5.

In one further preferred embodiment, the pharmaceutical composition comprises PTH at a concentration of 1.4 mg/ml, methionine at a concentration of 2 mg/ml, poloxamer 237 at a concentration of 0.3 mg/ml, and a physiologically acceptable buffering agent that maintains the pH of the formulation at a pH of 5.5.

In one further preferred embodiment, the pharmaceutical composition comprises PTH at a concentration of 1.4 mg/ml, methionine at a concentration of 2 mg/ml, poloxamer 237 at a concentration of 0.05 mg/ml, and a physiologically acceptable buffering agent that maintains the pH of the formulation at a pH of 5.5.A PTH formulation as described herein may include one or more tonicity modifiers. Materials that may be used as tonicity modifiers include, for example, sodium chloride, sucrose and polyol excipients, such as sorbitol, glycerol, and mannitol. In one embodiment, where the PTH formulation is to be lyophilized for storage and reconstituted prior to administration, a non-volatile buffer as described herein may be provided in combination with one or more tonicity modifiers that are selected such that the one or more tonicity modifiers co-lyophilize with the non-volatile buffer and the PTH to form an amorphous cake. In one embodiment, sodium chloride is used. Where sodium chloride is included as a tonicity modifier, sodium chloride may, for example, be provided at a concentration of 1 mg/ml to 10 mg/ml, 2 mg/ml to 5 mg/ml, 3 mg/ml to 4 mg/ml, or 3.5 mg/ml to 4 mg/ml. In one such embodiment, a PTH formulation as described herein may include 4.5 mg NaCl in a solution volume of 1.15 ml. Where mannitol is included as a tonicity modifier, mannitol may, for example, provided at a concentration of 15 mg/ml to 50 mg/ml, 20 mg/ml to 40 mg/ml, or 25 mg/ml to 30 mg/ml. In one such embodiment, a PTH formulation as described herein may include 30 mg of mannitol in a solution volume of 1.15 ml. In yet another embodiment, both sodium chloride and mannitol are included as tonicity modifiers. In such an embodiment, the sodium chloride and mannitol may, for example, be provided in the PTH formulation at the concentrations detailed above. In another embodiment, xylitol or sorbitol is used. Where sucrose, xylitol or sorbitol is included as a tonicity modifier, the sucrose, xylitol or sorbitol can for example be provided at a concentration of, for example, 1 mg/ml to 100 mg/ml, 50 mg/ml to 500 mg/ml, or 50 mg/ml to 300 mg/ml, such as 50 mg/ml to 100 mg/ml. In another embodiment, glycerol is used. Where glycerol is included as a tonicity modifier, the glycerol may, for example, be provided at a concentration of 1 mg/ml to 30 mg/ml, such as 15 mg/ml to 30 mg/ml.

One or more pharmaceutically acceptable preservatives may also be included in a PTH formulation as described herein. Examples of suitable preservatives include m-cresol, benzyl alcohol, benzoic acid, phenol, and EDTA, such as selected from cresol, benzyl alcohol and EDTA. In one preferred embodiment, m-cresol is used as the preservative. Where m-cresol is used as a preservative, it can for example be included at a concentration of 1 mg/ml to 5 mg/ml, 2 mg/ml to 4 mg/ml, or 3 mg/ml to 4mg/ml. In one embodiment, a PTH formulation as described herein may include 3.62 mg of m-cresol in a solution volume of 1.15 ml. Where benzyl alcohol is included as a preservative, it may be included at a concentration of 8 mg/ml to 12 mg/ml or 9 mg/ml to 11 mg/ml. In one embodiment, a PTH formulation as described herein may include benzyl alcohol as a preservative at a concentration of 10 mg/ml. Where EDTA is included as a preservative, it may be included at a concentration of up to 5 mg/ml, up to 4 mg/ ml, up to 3 mg/ml, up to 2 mg/ml, or up to 1 mg/ml. In one such embodiment, a PTH formulation as described herein may include EDTA at a concentration of 1 mg/ml.

One or more pharmaceutically acceptable stabilisers can be included in a PTH formulation as described herein. Examples of suitable stabilisers include xylitol, lysine, histidine and microcrystalline cellulose. In one embodiment, a PTH formulation as described herein may include xylitol at a concentration of 50 mg/ml to 300 mg/ml, such as, for example, 100 mg/ml to 300 mg/ml. In another embodiment, a PTH formulation as described herein may include lysine at a concentration of 1 mg/ml to 10 mg/ml, such as, for example, 2 mg/ml to 5 mg/ml. In another embodiment, a PTH formulation as described herein may include histidine at a concentration of 0.5 mg/ml to 5 mg/ml, such as, for example, 1 mg/ml to 5 mg/ml. In another embodiment, a PTH formulation as described herein may include microcrystalline cellulose at a concentration of 1 mg/ml to 1 S mg/ml, such as, for example, 2 mg/ml to 10 mg/ml. If the PTH formulation includes microcrystalline cellulose, the formulation may be in a form of a dispersion or a slurry.

A PTH formulation according to the present description may be prepared as a solution ready for administration. Alternatively, a PTH formulation according to the present description may be reconstituted from a lyophilized product. Where the PTH formulation is prepared by reconstituting a lyophilized product, the lyophilized product and reconstituting vehicle are prepared and combined in a manner that yields a solution formulation that includes formulation constituents at the concentrations described herein.

Where the PTH formulation is lyophilized prior to reconstitution, the lyophilized powder may be prepared from the lyophilization of a sterile, aqueous PTH solution. As is conventional in the art of formulation, the lyophilization process typically entails a temperature cycling process that is controlled carefully to ensure that drying proceeds uniformly and to substantial completion, for example, to yield a powder containing not more than about 2% water by weight. Lyophilization protocols suitable for producing a lyophilized powder that is ready for reconstitution are described in, for example, U.S. Patent 5,496,801 and U.S. Published App. No. 2005/0209144 A1, with the contents of both such references being incorporated herein by reference. Once a lyophilized powder is formed, the powder may be reconstituted in a suitable vehicle, such as a sterile water vehicle, using standard methods. If desired, the vehicle used to reconstitute the lyophilized powder can include one or more of the formulation constituents described herein. That is, where a PTH formulation as described herein is prepared through reconstitution of a lyophilized PTH composition, one or more formulation constituents may be absent from the lyophilized PTH composition but present in the aqueous vehicle used for reconstitution and formation of the PTH formulation to be administered. In one preferred embodiment, the pharmaceutical composition may be suitable for administration by injection.

The PTH formulations described herein may be used for therapeutic purposes, and particularly for the treatment of osteoporosis. Osteoporosis therapy entails administration of the reconstituted preparation by injection, such as sub-cutaneous injection, in unit doses that reflect the prescribed treatment regimen. In a preferred treatment regimen, the frequency of dosing of the PTH is from once weekly to thrice daily, such as once daily, twice daily, or thrice daily.

In one therapeutic embodiment, a PTH formulation as described herein is used in a method for treating osteoporosis in a human and the method includes administering a single injection of the PTH formulation in an amount that provides a dose of 25 µg PTH to 150 µg PTH. In another embodiment, a method for treating osteoporosis in a human includes administering a single injection of a PTH formulation as described herein in an amount that provides a dose of 25 µg PTH to 100 µg PTH. In yet another embodiment, a method for treating osteoporosis in a human includes administering a single injection of a PTH formulation as described herein in an amount that provides a dose of 25 µg PTH to 75 µg PTH. In still another embodiment, a method for treating osteoporosis in a human includes administering a single injection of a PTH formulation as described herein in an amount that provides a dose of 50 µg PTH to 75 µg PTH. In one preferred embodiment, the dosage is about 100 µg PTH. If the PTH used in the method for treating osteoporosis is an analogue, variant or homologue of hPTH(1-84), it is understood by one skilled in the art that PTH dosages giving a functionally equivalent effect to hPTH(1-84) at the above-mentioned dosages can also be employed.

In addition to therapeutic use, the present PTH formulations can be formulated and administered to aid in medical diagnosis, and particularly to assist in establishing the diagnosis of hypoparathyroidism and pseudohypoparathyoidism in hypocalcemic patients. Except for the dose of PTH, the composition of the PTH preparation will remain as described herein for therapeutic use. An intravenously infused, single dose of hPTH(1-84) or PTH bioequivalent that is equal to 200 International Units of PTH activity is appropriate for this diagnostic purpose. Diagnosis is then made by determining the effect of administered PTH on urinary cAMP levels, with cAMP elevation being indicative of the hypoparathyroidism condition, rather than its pseudoform.

Accordingly, in one embodiment, the pharmaceutical composition is administered by injection.

Further embodiments are:
1. A pharmaceutical composition comprising PTH and at least one non-ionic surfactant.
2. The composition according to embodiment 1, wherein the non-ionic surfactant is selected from a Poloxamer surfactant, a PVA surfactant, a Tween surfactant, Brij surfactant and a Triton-X surfactant.
3. The composition according to any of the preceding embodiments, wherein the non-ionic surfactant is a Poloxamer surfactant.
4. The composition according to any of the preceding embodiments, wherein the non-ionic surfactant is selected from Poloxamer 407, Poloxamer 338, Poloxamer 237, Poloxamer 188, and Poloxamer 124.
5. The composition according to any of the preceding embodiments, wherein the non-ionic surfactant is a Poloxamer surfactant provided at a concentration of up to 2 mg/ml.
6. The composition according to any of the preceding embodiments, wherein the non-ionic surfactant is a Poloxamer surfactant provided at a concentration selected from 0.02 mg/ml to 1 mg/ml, 0.05 mg/ml to 0.5 mg/ml, 0.05 mg/ml to 0.75 mg/ml, 0.05 mg/ml to 0.3 mg/ ml, 0.2 mg/ml to 0.5 mg/ml, 0.3 mg/ml to 0.5 mg/ml, and 0.3 mg/ml to 1 mg/ml.
7. The composition according to any of the preceding embodiments, wherein the non-ionic surfactant comprises a combination of two or more Poloxamer surfactants selected from Poloxamer 407, Poloxamer 338, Poloxamer 237; Poloxamer 188, and Poloxamer 124.
8. The composition according to embodiment 7, wherein each of the two or more Poloxamer surfactants is provided at a concentration selected from 0.02 mg/ml to 1 mg/ml, 0.05 mg/ml to 0.5 mg/ml, 0.05 mg/ml to 0.75 mg/ml, 0.05 mg/ml to 0.3 mg/ml, 0.2 mg/ml to 0.5 mg/ml, 0.3 mg/ml to 0.5 mg/ml, and 0.3 mg/ml to 1 mg/ml.
9. The composition according to any of the preceding embodiments, wherein the non-ionic surfactant is a PVA surfactant.
10. The composition according to embodiment 9, wherein the PVA surfactant is PVA 4-88.
11. The composition according to embodiment 9 or embodiment 10, wherein the PVA surfactant is provided at a concentration selected from 1 mg/ml to 15 mg/ml, 1 mg/ml to 10 mg/ml, 1 mg/ml to 7 mg/ml, 1 mg/ml to 5 mg/ml, 1 mg/ml to 3 mg/ml, 1 mg/ml to 2 mg/ml, 2 mg/ml to 3 mg/ml, 2 mg/ml to 10 mg/ml, 5 mg/ml to 15 mg/ml, 5 mg/ml to 10 mg/ml, 5 mg/ml to 7 mg/ml, 7mg/ml to 10 mg/ml, and 9 mg/ml to 10 mg/ml.
12. The composition according to any of the preceding embodiments, wherein the non-ionic surfactant is a Tween surfactant.
13. The composition according to embodiment 12, wherein the Tween surfactant is selected from Tween 20, Tween 40 and Tween 80.
14. The composition according to embodiment 12 or embodiment 13, wherein the Tween surfactant is provided at a concentration selected from a concentration selected from 0.02 mg/ml to 2 mg/ml and 0.02 mg/ml to 0.1 mg/ml.
15. The composition according to any of the preceding embodiments, wherein the non-ionic surfactant is a Brij surfactant.
16. The composition according to embodiment I5, wherein the Brij surfactant is selected from Brij 35 and Brij 52.
17. The composition according to embodiment 15 or embodiment 16, wherein said Brij surfactant is provided at a concentration selected from 0.05mg/ml to 2 mg/ml and 0.02 mg/ml to 0.1 mg/ml.
18. The composition according to any of the preceding embodiments, wherein the non-ionic surfactant is a Triton-X surfactant.
19. The composition according to embodiment 18, wherein the Triton surfactant is Triton X-100 provided at a concentration selected from 0.05 mg/ml to 2 mg/ml and 0.1 mg/ml to 0.3 mg/ml.
20. The composition according to any of the preceding embodiments, wherein the composition further comprises at least one antioxidant.
21. The composition according to embodiment 20, wherein the composition comprises an antioxidant selected from butylhydroxytoluen (BHT), butylhydroxyanisol (BHA), tocopherol (Vitamin E), propylgallate, ascorbic acid, sodium bisulfite, monothioglycerol, methionine and EDTA (sodium, dicalcium, anhydrous).
22. The composition according to embodiment 20 or embodiment 21, wherein the antioxidant is provided at a concentration selected from up to 1 mg/ml, 1 mg/ml to 10 mg/ml, 1 mg/ml to 5 mg/ml, 1 mg/ml to 4 mg/ml, 1 mg/ml to 3 mg/ml, 1 mg/ml to 2 mg/ml, 2 mg/ml to 9 mg/ml, 3 mg/ml to 8 mg/ml, 4 mg/ml to 7 mg/ml, or 5 mg/ml to 6 mg/ml, 10 mg/ml to 20 mg/ml, 11 mg/ml to 19 mg/ml, 12 mg/ml to 18 mg/ml, 13 mg/ml to 17 mg/ml, and 14 mg/ml to 16 mg/ml.
23. The composition according to any of embodiments 20-22, wherein the antioxidant is methionine.
24. The composition according to embodiment 23, wherein the methionine is provided at a concentration of up to 20 mg/ml.
25. The composition according to embodiment 23, wherein the methionine is provided at a concentration selected from 5 mg/ml, 1 mg/ml to 4 mg/ml, 2 mg/ml to 4 mg/ml, 2 mg/ml to 16 mg/ml, 15 mg/ml to 20 mg/ml, and 15 mg/ml to 17 mg/ml.
26. The composition according to any of the preceding embodiments, wherein the PTH is selected from hPTH(1-84) and a non-human, mammalian PTH molecule, as well as biologically active variants and homologs of hPTH(1-84) and non-human, mammalian PTH molecules.
27. The composition according to embodiment 26, wherein the composition includes a biologically active homolog of hPTH(1-84) sharing a sequence identity to hPTH(1-84) of at least 85%.
28. The composition according to embodiment 27, wherein the biologically active homolog shares a sequence identity to hPTH(1-84) of at least 95%.
29. The composition according to embodiment 27, wherein the biologically active homolog shares a sequence identity to hPTH(1-84) of at least 98%.
30. The composition according to any of the preceding embodiments, wherein the PTH is included at a concentration selected from 0.3 mg/ml to 10 mg/ml, 0.3 mg/ml to 5 mg/ml, 0.3 mg/ml to 3 mg/ml, 0.3 mg/ml to 2 mg/ml, 0.3 mg/ml to 1 mg/ml, and 0.4 mg/ml to 8 mg/ml.
31. The composition according to any of embodiments 1-29, wherein the PTH is included at a concentration selected from 1 mg/ml to 10 mg/ml, 1 mg/ml to 5 mg/ml, 1 mg/ml to 3 mg/ml, and 1 mg/ml to 2 mg/ml.
32. The composition according to any of embodiments 26-29, wherein the PTH is included at concentration selected from 0.3 mg/ml to 3 mg/ml, 1 mg/ml to 3 mg/ml, and 1 mg/ml to 2 mg/ml.
33. The composition according to any of the preceding embodiments, wherein said composition further comprises a pharmaceutically acceptable solvent.
34. The composition according to any of the preceding embodiments, further comprising a physiologically acceptable buffering agent.
35. The composition according to embodiment 34, wherein the physiologically acceptable buffering agent is selected from acetate, citrate, phosphate, TRIS (Tromethamine), arginine and carbonate buffers.
36. The composition according to embodiment 34 or embodiment 35, wherein the physiologically suitable buffering agent is a non-volatile buffering agent.
37. The composition according to any of the preceding embodiments, further comprising at least one tonicity modifier.
38. The composition according to embodiment 37, wherein at least one tonicity modifier is selected from sodium chloride, sucrose and a polyol excipient.
39. The composition according to embodiment 38, wherein the polyol excipient is selected from sorbitol, glycerol, and mannitol.
40. The composition according to any of embodiments 37-39, wherein the at least one tonicity modifier co-lyophilizes with the non-volatile buffering agent.
41. The composition according to any of embodiments 37-40, wherein the at least one tonicity modifier comprises sodium chloride provided at a concentration selected from 1 mg/ml to 10 mg/ml, 2 mg/ml to 5 mg/ml, 3 mg/ml to 4 mg/ml, and 3.5 mg/ml to 4 mg/ml.
42. The composition according to embodiment 37, wherein the at least one tonicity modifier comprises mannitol provided at a concentration selected from 15mg/ml to 50 mg/ml, 20 mg/ml to 40 mg/ml, and 25mg/ml to 30 mg/ml.
43. The composition according to embodiment 37, wherein the at least one tonicity modifier is selected from sucrose and sorbitol and is provided at a concentration selected from 1 mg/ml to 100 mg/ml, 50 mg/ml to 500 mg/ml, 50 mg/ml to 300 mg/ml, and 50 mg/ml to 100 mg/ml.
44. The composition according to embodiment 37, wherein the at least one tonicity modifier comprises glycerol provided at a concentration selected from 1 mg/ml to 30 mg/ml and 15 mg/ml to 30 mg/ml.
45. The composition according to any of embodiments 37-40, wherein the tonicity modifier comprises sodium chloride and mannitol, the sodium chloride is provided at a concentration selected from 1 mg/ml to 10 mg/ml, 2 mg/ml to 5 mg/ml, 3 mg/ml to 4 mg/ml, and 3.5 mg/ml to 4 mg/ml, and the mannitol is provided at a concentration selected from 15mg/ml to 50 mg/ml, 20 mg/ml to 40 mg/ml, and 25mg/ml to 30 mg/ml.
45. The composition according to any of the preceding embodiments, further comprising at least one pharmaceutically acceptable preservative.
46. The composition according to embodiment 45, wherein the at least one pharmaceutically acceptable preservative is selected from cresol, benzyl alcohol and EDTA.
47. The composition according to embodiment 46, wherein the at least one pharmaceutically acceptable preservative comprises m-cresol.
48. The composition of embodiment 47, wherein the m-cresol is provided at a concentration selected from 1 mg/ml to 5 mg/ml, 2 mg/ml to 4 mg/ml, and 3 mg/ml to 4mg/ml.
49. The composition according to embodiment 46, wherein the at least one pharmaceutically acceptable preservative comprises benzyl alcohol.
50. The composition of embodiment 49, wherein the benzyl alcohol is provided at a concentration selected from 8 mg/ml to 12 mg/ml and 9 mg/ml to 11 mg/ml.
51. The composition according to embodiment 46, wherein the at least one pharmaceutically acceptable preservative comprises EDTA provided at a concentration selected from up to 5 mg/ml, up to 4 mg/ ml, up to 3 mg/ml, up to 2 mg/ml, and up to 1 mg/ml.
52. The composition according to any of the preceding embodiments, further comprising at least one pharmaceutically acceptable stabilizer.
53. The composition according to embodiment 52, wherein the at least one pharmaceutically acceptable stabilizer is selected from xylitol, lysine, histidine and microcrystalline cellulose.
54. The composition according to embodiment 53, wherein the at least one pharmaceutically acceptable stabilizer comprises xylitol provided at a concentration selected from 50 mg/ml to 300 mg/ml and 100 mg/ml to 300 mg/ml.
55. The composition according to embodiment 53, wherein the at least one pharmaceutically acceptable stabilizer comprises lysine provided at a concentration selected from 1 mg/ml to 10 mg/ml and 2 mg/ml to 5 mg/ml.
56. The composition according to embodiment 53, wherein the at least one pharmaceutically acceptable stabilizer comprises histidine provided at a concentration selected from 0.5 mg/ml to 5 mg/ml and 1 mg/ml to 5 mg/ml.
57. The composition according to embodiment 53, wherein the at least one pharmaceutically acceptable stabilizer comprises microcrystalline cellulose provided at a concentration selected from 1 mg/ml to 15 mg/ml and 2 mg/ml to 10 mg/ml.
58. The composition according to any of the preceding embodiments, wherein said composition is lyophilized.
59. Method for preparing a pharmaceutical composition, comprising the steps of reconstituting the composition according to embodiment 58 into solution with a pharmaceutically acceptable vehicle.
60. The composition according to any of embodiments 1-58 for use as a medicament.
61. The composition according to any of embodiments 1-58 for use as a medicament for treatment or prevention of osteoporosis.

In the following, the present invention is illustrated by figures and examples which are not intended to limit the scope of the present invention.

### FIGURE LEGENDS

Figure 1: Turbidimetry measurements upon storage at 5°C.
Figure 2: RP-HPLC results showing the % main peak of PTH when stored at 5°C.
Figure 3: CE-HPLC results showing the % of the main peak of PTH when stored at 5°C. During the analysis an old column was used, which has resulted in bad separation of the peaks in the chromatogram. This is probably the reason why the results are higher after 24 month compared to after 18 month.

### EXAMPLES

The hPTH(1-84) used in the formulation studies was produced and excreted from a strain of Escherichia coli by known methods (See, e.g., U.S. Pat. No. 5,223,407, U.S. Pat. No. 5,646,015 and U.S. Pat. No. 5,629,205). Briefly, the human parathyroid hormone gene on plasmid pJT42 was fused with E. coli outer membrane protein A (ompA) secretion signal DNA. Also present on the plasmid is a lactose repressor gene (lacIq). Translation of the ompA-rhPTH mRNA and further processing by endogenous peptidase resulted in the production of mature 84 amino acid human parathyroid hormone which was harvested from the bacterial culture broth.

The recombinant expression was followed by purification of hPTH(1-84) to a preparation essentially free of contaminants. The purification process, which involved methods known in the art (See, e.g., U.S. Pat. No. 5,208,041), involved cell separation, filtration, ultrafiltration, ion exchange chromatography, hydrophobic interaction chromatography, preparative RP-HPLC, and a second ion exchange chromatography followed by desalting to yield a liquid bulk.

### Example 1

A reference PTH formulation was prepared. The reference formulation did not include a non-ionic surfactant or an antioxidant. The reference formulation was prepared by reconstituting a lyophilized PTH composition in a sterile aqueous vehicle. The lyophilized PTH composition included 1.61 mg hPTH(1-84), 4.5 mg sodium chloride, 30 mg mannitol, and 1.26 mg citric acid monohydrate. The sterile aqueous vehicle included sterile water prepared for injection and 3.15 mg/ml m-cresol. The lyophilized PTH composition was reconstituted in 1.15 ml of the sterile aqueous vehicle to provide a PTH formulation having a pH of 5.5 and including 1.4 mg/ml hPTH(1-84).

This reference formulation was stored at 25° C for 60 days, and the stability of the hPTH(1-84)included in the reference formulation was analyzed by RP-HPLC and CE-HPLC. The results of the RP-HPLC analysis are provided in Table l. The results of the CE-HPLC analysis are provided in Table 2.

**Table 1**

| **RP-HPLC** | **Time** **[days]** | **Reference** | |
|---|---|---|---|
| | | **1A** | **1B** |
| | 0 | 99.28 | 99.18 |
| **% main peak of PTH** | 15 | 95.60 | 95.26 |
| | 30 | 91.17 | 90.20 |
| | 60 | 76.32 | 78.82 |

**Table 2**

| **CE-HPLC** | **Time** **[days]** | **Reference** | |
|---|---|---|---|
| | | **1A** | **1B** |
| | 0 | 100 | 100 |
| **% main peak of PTH** | 15 | 92.45 | 92.03 |
| | 30 | 90.18 | 88.67 |
| | 60 | 74.18 | 71.06 |

### Example 2

Formulations including a non-ionic surfactant were prepared. These formulations were prepared according to Example 1, except that a non-ionic surfactant was included in the sterile aqueous vehicle used to reconstitute the lyophilized PTH composition. The formulations prepared according to this example exhibited a pH of 5.5.

Two different formulations including Poloxamer 188 were prepared. The first included Poloxamer 188 in the sterile aqueous vehicle at a concentration of 1.0 mg/ml. The second included Poloxamer 188 in the sterile aqueous vehicle at a concentration of 0.3 mg/ml.

Two different formulations included Poloxamer 407 were also prepared. The first included Poloxamer 407 in the sterile aqueous vehicle at a concentration of 1.0 mg/ml. The second included Poloxamer 407 in the sterile aqueous vehicle at a concentration of 0.3 mg/ml.

The formulations including Poloxamer 188 and Poloxamer 407 were stored at 25° C for 60 days, and the stability of the hPTH(1-84) included in each formulation was analyzed using RP-HPLC and CE-HPLC. The results of the RP-HPLC analysis are provided in Table 3. The results of the CE-HPLC analysis are provided in Table 4. As can be seen from Table 3 and Table 4, Poloxamer 188 and Poloxamer 407 both had a stabilizing effect on the hPTH(1-84) included in the formulations

**Table 3**

| **RP-HPLC % Change in Main Peak of PTH When Stored at 25°C** | | | | |
|---|---|---|---|---|
| **Time [days]** | **Poloxamer 188** | | **Poloxamer 407** | |
| | 1.0 mg/ml | 0.3 mg/ml | 1.0 mg/ml | 0.3 mg/ml |
| 0 | 100 | 100 | 99.8 | 100 |
| 15 | 97.4 | 97.81 | 97.46 | 97.46 |
| 30 | 95.06 | 94.15 | 95.23 | 94.86 |
| 60 | 90.05 | 89.98 | 74.80 | 76.81 |

**Table 4**

| **CE-HPLC % Change in Main Peak of PTH When Stored at 25°C** | | | | |
|---|---|---|---|---|
| **Time [days]** | **Poloxamer 188** | | **Poloxamer 407** | |
| | 1.0 mg/ml | 0.3 mg/ml | 1.0 mg/ml | 0.3 mg/ml |
| 0 | 100 | 100 | 100 | 100 |
| 15 | 99.68 | 99.66 | 99.70 | 99.68 |
| 30 | 96.67 | 96.95 | 96.96 | 97.16 |
| 60 | 87.27 | 88.28 | 79.15 | 78.72 |

### Example 3

A formulation including Poloxamer 188 and methionine was prepared. The formulation was prepared according to Example 1, except that 0.3 mg/ml Poloxamer 188 and 2 mg/ml methionine were included in the sterile aqueous vehicle used to reconstitute the lyophilized PTH composition. The formulation prepared according to this example exhibited a pH of 5.5.

The formulation including Poloxamer 188 and methionine was stored at 25° C for 60 days, and the stability of the hPTH(1-84) included this formulation was analyzed using RP-HPLC and CE-HPLC. The results of these analyses are provided in Table 5 and Table 6. As can be seen in the referenced tables, the combination of Poloxamer 188 and methionine included in the PTH formulation had a stabilizing effect on hPTH(1-84).

**Table 5**

| **RP-HPLC % Change in Main Peak of PTH When Stored at 25°C** | |
|---|---|
| **Time [days]** | **Methionine +** |
| | **Poloxamer 188** |
| | 2mg/ml + |
| | 0.3 mg/ml |
| 0 | 100 |
| 15 | 98.49 |
| 30 | 96.80 |
| 60 | 92.91 |

**Table 6**

| **CE-HPLC % Change in Main Peak of PTH When Stored at 25°C** | |
|---|---|
| **Time [days]** | **Methionine +** |
| | **Poloxamer 188** |
| | 2mg/ml + |
| | 0.3 mg/ml |
| 0 | 100 |
| 15 | 99.69 |
| 30¹ | 96.70 |
| 60 | 87.38 |

### Example 4

Two formulations including Poloxamer 407 and methionine were prepared. These formulations were prepared according to Example 1, except that Poloxamer 407 and methionine were included in the sterile aqueous vehicle used to reconstitute the lyophilized PTH composition. The first formulation was prepared by including 0.3 mg/ml Poloxamer 407 and 2 mg/ml methionine in the sterile aqueous vehicle used to reconstitute the lyophilized PTH composition. The second formulation was prepared by including 0.3 mg/ml Poloxamer 407 and 6 mg/ml methionine in the sterile aqueous vehicle used to reconstitute the lyophilized PTH composition. The formulations prepared according to this example exhibited a pH of 5.5.

The formulations including Poloxamer 407 and methionine were stored at 25° C for 60 days, and the stability of the hPTH(1-84) included in each formulation was analyzed using RP-HPLC and CE-HPLC. The results of these analyses are provided in Table 7 & Table 8. As can be seen in the referenced tables, the combination of Poloxamer 407 and methionine included in the PTH formulations had a stabilizing effect on hPTH(1-84).

**Table 7**

| **RP-HPLC** | **Time [days]** | **Methionine 2mg/ml + Poloxamer 407 0.3mg/ml** | | **Methionine 6mg/ml + Poloxamer 407 0.3mg/ml** | |
|---|---|---|---|---|---|
| | | **2A** | **28** | **3A** | **3B** |
| | 0 | 100 | 99.51 | 100 | 100 |
| **% main peak of PTH** | 15 | 98.53 | 98.54 | 98.51 | 98.74 |
| | 30 | 96.61 | 96.56 | 96.12 | 96.14 |
| | 60 | 92.78 | 93.41 | 91.57 | 92.78 |

**Table 8**

| **CE-HPLC** | **Time [days]** | **Methionine 2mg/ml + Poloxamer 407 0.3mg/ml** | | **Methionine 6mg/ml + Poloxamer 407 0.3mg/ml** | |
|---|---|---|---|---|---|
| | | **2A** | **2B** | **3A** | **3B** |
| | 0 | 100 | 100 | 100 | 100 |
| **% main peak of PTH** | 15 | 97.71 | 97.74 | 97.74 | 97.70 |
| | 30 | 96.00 | 95.92 | 96.00 | 95.62 |
| | 60 | 85.32 | 85.86 | 85.27 | 85.13 |

### Example 5

Two formulations including Poloxamer 237 and methionine were prepared. These formulations were prepared according to Example 1, except that Poloxamer 237 and methionine were included in the sterile aqueous vehicle used to reconstitute the lyophilized PTH composition. The first formulation was prepared by including 0.3 mg/ml Poloxamer 237 and 2 mg/ml methionine in the sterile aqueous vehicle used to reconstitute the lyophilized PTH composition. The second formulation was prepared by including 0.05 mg/ml Poloxamer 237 and 2 mg/ml methionine in the sterile aqueous vehicle used to reconstitute the lyophilized PTH composition. The formulations prepared according to this example exhibited a pH of 5.5.

The formulations including Poloxamer 237 and methionine were stored at 25° C for 60 days, and the stability of the hPTH(I-84) included in each formulation was analyzed using RP-HPLC and CE-HPLC. The results of these analyses are provided in Table 9 & Table 10. As can be seen in the referenced tables, the combination of Poloxamer 237 and methionine included in the PTH formulations had a stabilizing effect on hPTH(1-84).

**Table 9**

| **RP-HPLC** | **Time [days]** | **Methionine 2mg/ml + Poloxamer 237 0.3mg/ml** | | **Methionine 2mg/ml + Poloxamer 237 0.05mg/ml** | |
|---|---|---|---|---|---|
| | | **7A** | **7B** | **8A** | **8B** |
| | 0 | 99.75 | 99.51 | 99.77 | 99.77 |
| **% main peak of PTH** | 15 | 98.52 | 98.23 | 98.33 | 97.89 |
| | 30 | 96.07 | 96.17 | 96.77 | 95.09 |
| | 60 | 93.16 | 93.73 | 92.95 | 93.10 |

**Table 10**

| **CE-HPLC** | **Time [days]** | **Methionine 2mg/ml + Poloxamer 237 0.3mg/ml** | | **Methionine 2mg/ml + Poloxamer 237 0.05mg/ml** | |
|---|---|---|---|---|---|
| | | **7A** | **7B** | **8A** | **8B** |
| **% main peak of PTH** | 0 | 100 | 100 | 100 | 100 |
| | 15 | 97.80 | 97.71 | 97.81 | 97.78 |
| | 30 | 96.31 | 96.26 | 96.17 | 95.91 |
| | 60 | 85.64 | 85.26 | 85.10 | 85.69 |

### Example 6

Two formulations including Poloxamer 338 and methionine were prepared. These formulations were prepared according to Example 1, except that Poloxamer 338 and methionine were included in the sterile aqueous vehicle used to reconstitute the lyophilized PTH composition. The first formulation was prepared by including 0.3 mg/ml Poloxamer 338 and 2 mg/ml methionine in the sterile aqueous vehicle used to reconstitute the lyophilized PTH composition. The second formulation was prepared by including 1 mg/ml Poloxamer 338 and 2 mg/ml methionine in the sterile aqueous vehicle used to reconstitute the lyophilized PTH composition. The formulations prepared according to this example exhibited a pH of 5.5.

The formulations including Poloxamer 338 and methionine were stored at 25° C for 60 days, and the stability of the hPTH(1-84) included in each formulation was analyzed using RP-HPLC and CE-HPLC. The results of these analyses are provided in Table 11 & Table 12. As can be seen in the referenced tables, the combination of Poloxamer 338 and methionine included in the PTH formulations had a stabilizing effect on hPTH(1-84).

**Table 11**

| **RP-HPLC** | **Time [days]** | **Methionine 2mg/ml + Poloxamer 338 0.3mg/ml** | | **Methionine 2mg/ml + Poloxamer 338 1mg/ml** | |
|---|---|---|---|---|---|
| | | **9A** | **9B** | **10A** | **10B** |
| **% main peak of PTH** | 0 | 99.48 | 99.48 | 99.54 | 99.50 |
| | 15 | 98.52 | 98.46 | 98.18 | 97.85 |
| | 30 | 96.44 | 97.13 | 97.33 | 96.50 |
| | 60 | 93.19 | 93.07 | 93.21 | 93.13 |

**Table 12**

| **CE-HPLC** | **Time [days]** | **Methionine 2mg/ml + Poloxamer 338 0.3mg/ml** | | **Methionine 2mg/ml + Poloxamer 338 1mg/ml** | |
|---|---|---|---|---|---|
| | | **9A** | **9B** | **10A** | **10B** |
| | 0 | 100 | 100 | 100 | 100 |
| **% main peak of PTH** | 15 | 97.79 | 97.69 | 97.82 | 97.83 |
| | 30 | 96.10 | 96.26 | 96.26 | 96.36 |
| | 60 | 83.89 | 85.67 | 85.02 | 85.61 |

### Example 7

Two formulations including PVA 4-88 and methionine were prepared. These formulations were prepared according to Example 1, except that PVA 4-88 and methionine were included in the sterile aqueous vehicle used to reconstitute the lyophilized PTH composition. The first formulation was prepared by including 10 mg/ml PVA 4-88 and 2 mg/ml methionine in the sterile aqueous vehicle used to reconstitute the lyophilized PTH composition. The second formulation was prepared by including 7 mg/ml PVA 4-88 and 2 mg/ml methionine in the sterile aqueous vehicle used to reconstitute the lyophilized PTH composition. The formulations prepared according to this example exhibited a pH of 5.5.

The formulations including PVA 4-88 and methionine were stored at 25° C for 60 days, and the stability of the hPTH(1-84) included in each formulation was analyzed using RP-HPLC and CE-HPLC. The results of these analyses are provided in Table 13 & Table 14. As can be seen in the referenced tables, the combination of PVA 4-88 and methionine included in the PTH formulations had a stabilizing effect on hPTH(1-84).

**Table 13**

| **RP-HPLC** | **Time [days]** | **Methionine 2mg/ml + PVA 4-88 10mg/ml** | | **Methionine 2mg/ml + PVA 4-88 7mg/ml** | |
|---|---|---|---|---|---|
| | | **11A** | **11B** | **12A** | **12B** |
| | 0 | 99.77 | 99.56 | 99.50 | 99.46 |
| **% main peak of PTH** | 15 | 97.94 | 98.14 | 97.92 | 97.91 |
| | 30 | 97.10 | 98.04 | 96.86 | 96.76 |
| | 60 | 93.11 | 93.10 | 90.37 | 92.86 |

**Table 14**

| **CE-HPLC** | **Time [days]** | **Methionine 2mg/ml + PVA 4-88 10mg/ml** | | **Methionine 2mg/ml + PVA 4-88 7mg/ml** | |
|---|---|---|---|---|---|
| | | **11A** | **118** | **12A** | **12B** |
| | 0 | 100 | 100 | 100 | 100 |
| **% main peak of PTH** | 15 | 97.85 | 97.89 | 97.91 | 97.89 |
| | 30 | 96.37 | 96.41 | 96.39 | 96.25 |
| | 60 | 85.58 | 85.33 | 84.91 | 85.63 |

### Example 8

Formulations including different Poloxamer surfactants and methionine were prepared. These formulations were prepared according to Example 1, except that either Poloxamer 407, Poloxamer 237, Poloxamer 338, or Poloxamer 188 and methionine were included in the sterile aqueous vehicle used to reconstitute the lyophilized PTH composition. The formulation was prepared by including 0.3 mg/ml Poloxamer surfactant and 2 mg/ml methionine in the sterile aqueous vehicle used to reconstitute the lyophilized PTH composition. The formulations prepared according to this example exhibited a pH of 5.5.

The formulations including Poloxamer surfactant and methionine were stored at 25° C for 60 days, and the stability of the hPTH(1-84) included in each formulation was analyzed using RP-HPLC (Table 15) and CE-HPLC (Table 16). As can be seen in the referenced tables, the combination of a Poloxamer surfactant and methionine included in the PTH formulations had a stabilizing effect on hPTH(1-84), independent from the type of Poloxamer which have been added.

**Table 15**

| **Time [days]** | **Reference** | | **Methionine + Poloxamer 407** | | | **Methionine + Poloxamer 237** | | **Methionine + Poloxamer 338** | **Methionine + Poloxamer 188** | |
|---|---|---|---|---|---|---|---|---|---|---|
| | | | 2mg/ml + 0.3 mg/ml | | | 2mg/ml + 0.3 mg/ml | | 2mg/ml + 0.3 mg/ml | 2m/ml + 0.3 mg/ml | |
| Exp. No. | PTH-07 | | PTH-04 | PTH-05 | PTH-07 | PTH-07 | PTH-10 | PTH-07 | PTH-04 | PTH-10 |
| 0 | 99.28 | 99.18 | 100 | 100 | 99.51 | 99.75 | 99.72 | 99.48 | 100 | 99.73 |
| 15 | 95.60 | 95.26 | 98.42 | 98.37 | 98.54 | 98.52 | 98.18 | 98.52 | 98.49 | 97.92 |
| 30 | 91.17 | 90.20 | 96.65 | 96.64 | 96.56 | 96.07 | 96.32 | 96.44 | 96.80 | 95.69 |
| 60 | 76.32 | 78.82 | 92.30 | 94.61 | 93.41 | 93.16 | 88.91 | 93.19 | 92.91 | 88.36 |

**Table 16**

| **Time [days]** | **Reference** | | **Methionine + Poloxamer 407** | | | **Methionine + Poloxamer 237** | | **Methionine + Poloxamer 338** | **Methionine + Poloxamer 188** | |
|---|---|---|---|---|---|---|---|---|---|---|
| | | | 2mg/ml + 0.3 mg/ml | | | 2mg/ml + 0.3 mg/ml | | 2mg/ml + 0.3 mg/ml | 2mg/ml + 0.3 mg/ml | |
| Exp. no. | PTH-07 | | PTH-04 | PTH-05 | PTH-07 | PTH-07 | PTH-10 | PTH-07 | PTH-04 | PTH-10 |
| 0 | 100 | 100 | 100 | 100 | 100 | 100 | 99.73 | 100 | 100 | 100 |
| 15 | 92.45 | 92.03 | 96.67 | 97.47 | 97.71 | 97.80 | 99.05 | 97.79 | 99.69 | 98.99 |
| 30 | 90.18 | 88.67 | 97.25 | 94.25 | 96.00 | 96.31 | 93.79 | 96.10 | 96.70 | 93.67 |
| 60 | 74.18 | 71.06 | 87.97 | 87.46 | 85.32 | 85.64 | 86.58 | 83.89 | 87.38 | 87.22 |

It is surprising that even though the molecular weight of the different poloxamers are very different (ranging from 7835 to 15050 g/mol; Poloxamer 237 ≤ Poloxamer 188 < Poloxamer 407 ≤ Poloxamer 338; see Table 17), and in addition the concentration of the poloxamers added are the same (0.3 mg/ml), the different formulations still have the same chemical stabilising effect of PTH.

**Table 17**

| **Poloxamer** | **Molecular weight range** | **Average molecular weight** |
|---|---|---|
| | **Ig/moll** | **[g/mol]** |
| Poloxamer 188 | 7680-9510 | 8595 |
| Poloxamer 237 | 6840-8830 | 7835 |
| Poloxamer 338 | 12700-17400 | 15050 |
| Poloxamer 407 | 9840-14600 | 12220 |

### Example 9

Formulations including different Poloxamer surfactants and methionine were prepared. These formulations were prepared according to Example 1, except that either Poloxamer 407, Poloxamer 237, or Poloxamer 338 and methionine were included in the sterile aqueous vehicle used to reconstitute the lyophilized PTH composition. The formulation was prepared by including 0.3 mg/ml Poloxamer surfactant and 2 mg/ml methionine in the sterile aqueous vehicle used to reconstitute the lyophilized PTH composition. The formulations prepared according to this example exhibited a pH of 5.5.

The formulations including Poloxamer surfactant and methionine were stored at 25° C for 147 hours, and the physical stability of the hPTH(1-84) included in each formulation was analyzed by visual determination upon shaking (Table 18). The results show that the formulation containing Poloxamer 237 is improving the physical stability of PTH the most, while the formulation with Poloxamer 407 has the least stabilising effect. It is surprising that the physical stabilising effect of PTH is higher for the formulation containing Poloxamer 338 compared to Poloxamer 407 even though they have a similar molecular weight.

**Table 18**

| **Time [hours]** | **Methionine 2mg/ml + Poloxamer 407 0.3mg/ml** | | **Methionine 2mg/ml** + **Poloxamer 237 0.3 mg/ml** | | **Methionine 2mg/ml + Poloxamer 338 0.3mg/ml** | |
|---|---|---|---|---|---|---|
| | **A** | **B** | **A** | **B** | **A** | **B** |
| 0 | Clear | Clear | Clear | Clear | Clear | Clear |
| 7.5 | Clear | Clear | Clear | Clear | Clear | Clear |
| 24 | A bit unclear | A bit unclear | Clear | Clear | Clear | Clear |
| 31.5 | A bit unclear | A bit unclear | Clear | Clear | Clear | Clear |
| 47.5 | Unclear | A bit unclear | Clear | Clear | Clear | Clear |
| 55.5 | Unclear | A bit unclear | Clear | Clear | A bit unclear | A bit unclear |
| 73.5 | Unclear | A bit unclear | Clear | Clear | A bit unclear | A bit unclear |
| 80 | Unclear | A bit unclear | Clear | Clear | A bit unclear | A bit unclear |
| 147 | Unclear | Unclear | A bit unclear | A bit unclear | A bit unclear | A bit unclear |

### Example 10

Formulations including cysteine or methionine were prepared. These formulations were prepared according to Example 1, except that two formulations included cysteine and two formulations included methionine in the sterile aqueous vehicle used to reconstitute the lyophilized PTH composition. The formulations were prepared by including either 2 mg/ml or 16 mg/ml cysteine, or by including either 2 mg/ml or 8 mg/ml methionine in the sterile aqueous vehicle used to reconstitute the lyophilized PTH composition.

The formulations including cysteine or methionine were stored at 25° C for 11 days, and the chemical stability of the hPTH(1-84) included in each formulation was analyzed by RP-HPLC (Table 19). Compared to the reference, only methionine exhibited a chemical stabilising effect of PTH.

**Table 19**

| **RP-HPLC** | **Time [days]** | **Reference** | **Cysteine** | | Reference | **Methionine** | |
|---|---|---|---|---|---|---|---|
| | | | <16 mg/ml | <2 mg/ml | | 2 mg/ml | 8 mg/ml |
| **% main peak of PTH** | 0 | 99.1 | 98.42 | 98.18 | 99.67 | 99.7 | 99.7 |
| | 2 | - | - | - | 98.9 | 99.57 | 99.56 |
| | 4 | - | - | - | 97.65 | 98.98 | 99.03 |
| | 6 | 92.91 | 89.17 | 66.08 | - | - | - |
| | 11 | 86.36 | 77.54 | 40.04 | 92.7 | 98.23 | 97.99 |

### Example 11

Formulations have been prepared according to the formulations as indicated in Table 20 below. These formulations were prepared according to Example 1, except that Poloxamer 407 and methionine were included in the sterile aqueous vehicle used to reconstitute the lyophilized PTH composition. The formulation was prepared by including 0.3 mg/ml Poloxamer 407 and 2 mg/ml methionine in the sterile aqueous vehicle used to reconstitute the lyophilized PTH composition. The formulations prepared according to this example exhibited either a pH of 5.5, or a pH of 4.

**Table 20**

| **Formulation candidate no.** | **Additive** | **Conc. [mg/ml]** | **pH** |
|---|---|---|---|
| 1 | Reference | - | 5.5 |
| 2 | Reference | - | 4 |
| 3 | Methionine | 2 | 5.5 |
| | Poloxamer 407 | 0.3 | |
| 4 | Methionine | 2 | 4 |
| | Poloxamer 407 | 0.3 | |

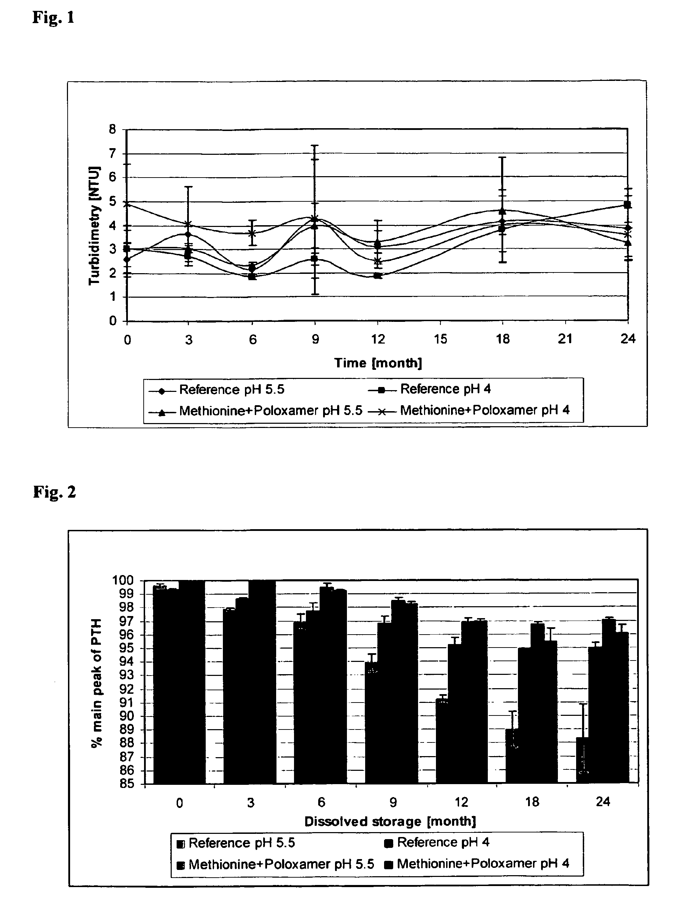

The formulations were stored at 5° C for 2 years, and the physical stability of hPTH(1-84) included in each formulation was determined by visual inspections and by turbidimetry measurements, while the chemical stability was determined by RP-HPLC and CE-HPLC. Figure 1 shows the turbidimetry measurements obtained upon storage at 5°C for 2 years. The results presented in figure 1 show that all the formulations are within specifications during the 2 years of storage. In addition the results show that the turbidimetry response approximately the same for all four formulation candidates, which show that the physical stability of PTH is not improved by changing the formulation and/or pH.

The visual determinations obtained upon storage at 5°C for 2 years are presented in at 5°C and that no differences are observed between the formulations. As for the turbidimetry measurements these results also indicate that the physical stability of PTH is not improved when changing the formulation and/or pH.

**Table 21**

| **Time [months]** | **Reference, pH 5.5** | | | **Reference, pH4** | | |
|---|---|---|---|---|---|---|
| | **1A** | **1B** | **1C** | **2A** | **2B** | **2C** |
| 0 | Clear | Clear | Clear | Clear | Clear | Clear |
| 3 | Clear | Clear | Clear | Clear | Clear | Clear |
| 6 | Clear | Clear | Clear | Clear | Clear | Clear |
| 9 | Clear | Clear | Clear | Clear | Clear | Clear |
| 12 | Clear | Clear | Clear | Clear | Clear | Clear |
| 18 | A bit unclear | A bit unclear | A bit unclear | A bit unclear | A bit unclear | A bit unclean |
| 24 | A bit unclear | A bit unclear | A bit unclear | A bit unclear | A bit unclear | A bit unclear |

**Table 22**

| **Time [months]** | **2mg/ml Methionine** + **0.03mg/ml Poloxamer 407, pH 5.5** | | | **2mg/ml Methionine** + **0.03mg/ml Poloxamer 407, pH** 4 | | |
|---|---|---|---|---|---|---|
| | **3A** | **3B** | **3C** | **4A** | **4B** | **4C** |
| 0 | Clear | Clear | Clear | Clear | Clear | Clear |
| 3 | Clear | Clear | Clear | Clear | Clear | Clear |
| 6 | Clear | Clear | Clear | Clear | Clear | Clear |
| 9 | Clear | Clear | Clear | Clear | Clear | Clear |
| 12 | Clear | Clear | Clear | Clear | Clear | Clear |
| 18 | A bit | A bit | A bit | A bit | A bit | A bit |
| | unclear | unclear | unclear | unclear | unclear | unclear |
| 24 | A bit | A bit | A bit | A bit | A bit | A bit |
| | unclear | unclear | unclear | unclear | unclear | unclear |

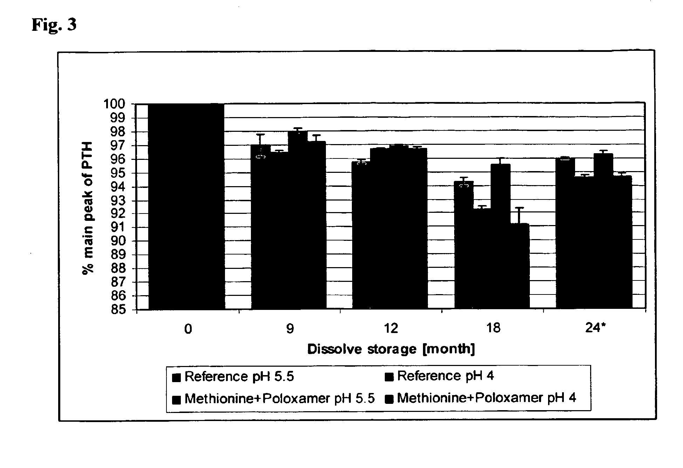

Figure 2 and 3 indicate the results obtained for RP-HPLC and CE-HPLC after 2 years of storage at 5°C. In figure 2 and 3 the obtained HPLC results generally show that the highest % main peak of PTH is observed for the formulation added methionine and Poloxamer 407 at pH 5.5.

After 2 years of storage at 5°C the RP-HPLC results show that the % main peak of PTH for the formulation with methionine and Poloxamer 407, pH 5.5 vs. the reference are 97.2% and 88.3% respectively. For the CE-HPLC results after 18 month of storage the two formulations are 95.5% and 94.6% respectively. These results indicate that the improvement of the chemical stability of PTH is significant for the RP-HPLC method, while a small improvement is observed for the CE-HPLC method. Due to the addition of methionine it is also expected that the improvement would be more pronounced for the RP-HPLC results.

The RP-HPLC results also show that the formulation including methionine and Poloxamer 407 at pH 5.5 is within specifications after 2 years of storage (% main peak of PTH ≥ 95%). For the CE-HPLC results the formulation is within specifications after 18 month of storage (% main peak of PTH ≥ 95%).

From the turbidimetry measurements and visual determinations it can be concluded that the physical stability of PTH is not improved by adding methionine and poloxamer 407 and/or by changing the pH. However, regarding the chemical stability of PTH it can be concluded that it is significantly improved when adding 2mg/ml methionine and 0.3mg/ml Poloxamer 407 and by keeping the pH at 5.5. In addition it can be concluded that the liquid formulation comprising 2mg/ml methionine and 0.3mg/ml Poloxamer 407 at pH 5.5 is stable and within specifications (% main peak of PTH ≥ 95%) for at least 18 month at 5°C.

### REFERENCES

US 5,208,041
US 5,223,407
US 5,646,015
US 5,629,205
EP-B-0383751
US 5,496,801
US 2005/0209144 A1
WO 2005/027978
EP 1 598 074 A1
WO 2004/007520 A2
WO 2005/028516 A2
WO 2006/017852 A2
Kimura et al. (1983) Biochem Biophys Res Comm, 114 (2):493.
Rodan et al. (1983) J. Clin. Invest. 72: 1511.
Rabbani et al. (1988) Encrinol. 123: 2709.
Keutmann et al., Current Research on Calcium Regulating Hormones, Cooper, C.W. (ed.), 1987, University of Texas Press, Austin, pp 57-63.
Fiedler, Lexikon der Hilfsstoffe, 5th Ed., Vol. 2, p. 1334.

## Claims

1. A pharmaceutical composition comprising, when dissolved in a suitable vehicle, PTH at a concentration of 1 mg/ml to 10 mg/ml, methionine provided at a concentration of 1 mg/ml to 10 mg/ml, at least one Poloxamer surfactant provided at a concentration from 0.02 mg/ml to 1 mg/ml, and a physiologically acceptable buffering agent that maintains the pH of the formulation at a pH of 5 to 6.5.

2. The composition according to claim 1, wherein the composition is in the form of a stable solution formulation.

3. The composition according to claim 1 or 2, wherein the at least one Poloxamer surfactant is selected from Poloxamer 188, Poloxamer 407, Poloxamer 237, Poloxamer 338, and Poloxamer 124.

4. The composition according to any of the preceding claims, wherein the PTH is selected from hPTH(1-84) and a non-human, mammalian PTH molecule, as well as biologically active variants and homologs of hPTH(1-84) and non-human, mammalian PTH molecules.

5. The composition according to claim 4, wherein the composition includes a biologically active homolog of hPTH(1-84) sharing a sequence identity to hPTH(1-84) of at least 85%, preferably wherein the biologically active homolog shares a sequence identity to hPTH(1-84) of at least 95%, more preferably wherein the biologically active homolog shares a sequence identity to hPTH(1-84) of at least 98%.

6. The composition of any of claims 1 to 5, comprising PTH at a concentration of 1.4 mg/ml, methionine at a concentration of 2 mg/ml, a poloxamer at a concentration of 0.3 mg/ml, and a physiologically acceptable buffering agent that maintains the pH of the formulation at a pH of 5.5.

7. The composition according to any of the preceding claims, wherein said composition further comprises a pharmaceutically acceptable solvent.

8. The composition according to any of the preceding claims, wherein the physiologically acceptable buffering agent is selected from citrate, acetate, phosphate, TRIS (Tromethamine), arginine and carbonate buffers.

9. The composition according to claim 8, wherein the physiologically suitable buffering agent is a non-volatile buffering agent.

10. The composition according to any of the preceding claims, further comprising at least one tonicity modifier, preferably wherein the at least one tonicity modifier is selected from sodium chloride, sucrose and a polyol excipient.

11. The composition according to claim 10, wherein the polyol excipient is selected from sorbitol, glycerol, and mannitol.

12. The composition according to any of claims 10 to 11, wherein the at least one tonicity modifier co-lyophilizes with the non-volatile buffering agent.

13. The composition according to any of the preceding claims, further comprising at least one pharmaceutically acceptable preservative, preferably wherein the at least one pharmaceutically acceptable preservative is selected from cresol, benzyl alcohol and EDTA.

14. The composition according to any of the preceding claims, further comprising at least one pharmaceutically acceptable stabilizer, preferably wherein the at least one pharmaceutically acceptable stabilizer is selected from xylitol, lysine, histidine and microcrystalline cellulose.

15. The composition according to any of the preceding claims, wherein said composition is suitable for administration by injection.

16. The composition according to any of the preceding claims, wherein said composition is lyophilized.

17. Method for preparing a pharmaceutical composition, comprising the steps of reconstituting the composition according to claim 16 into solution with a pharmaceutically acceptable vehicle.

18. The composition according to any of claims 1 to 16 for use as a medicament.

19. The composition according to any of claims 1 to 16 for use as a medicament for treatment or prevention of osteoporosis.

## Patentansprüche

1. Pharmazeutische Zusammensetzung umfassend, wenn sie in einem geeigneten Vehikel gelöst ist, PTH in einer Konzentration von 1 mg/ml bis 10 mg/ml, Methionin, das in einer Konzentration von 1 mg/ml bis 10 mg/ml bereitgestellt ist, mindestens ein Poloxamer-oberflächenaktives Mittel, das in einer Konzentration von 0,02 mg/ml bis 1 mg/ml bereitgestellt ist, und ein physiologisch verträgliches Puffermittel, das den pH der Formulierung bei einem pH von 5 bis 6,5 hält.

2. Zusammensetzung nach Anspruch 1, wobei die Zusammensetzung in der Form einer stabilen Lösungsformulierung ist.

3. Zusammensetzung nach Anspruch 1 oder 2, wobei das mindestens eine Poloxameroberflächenaktive Mittel aus Poloxamer 188, Poloxamer 407, Poloxamer 237, Poloxamer 338 und Poloxamer 124 ausgewählt ist.

4. Zusammensetzung nach einem beliebigen der vorangehenden Ansprüche, wobei das PTH aus hPTH(1-84) und einem nicht-humanen Säuger-PTH-Molekül, sowie biologisch wirksamen Varianten und Homologen von hPTH(1-84) und nicht-humanen Säuger-PTH-Molekülen ausgewählt ist.

5. Zusammensetzung nach Anspruch 4, wobei die Zusammensetzung ein biologisch wirksames Homolog von hPTH(1-84) einschließt, das mit hPTH(1-84) eine Sequenzidentität von mindestens 85% teilt, bevorzugt wobei das biologisch wirksame Homolog eine Sequenzidentität mit hPTH(1-84) von mindestens 95% teilt, weiter bevorzugt wobei das biologisch wirksame Homolog mit hPTH(1-84) eine Sequenzidentität von mindestens 98% teilt.

6. Zusammensetzung nach einem beliebigen der Ansprüche 1 bis 5, umfassend PTH in einer Konzentration von 1,4 mg/ml, Methionin in einer Konzentration von 2 mg/ml, ein Poloxamer in einer Konzentration von 0,3 mg/ml, und ein physiologisch verträgliches Puffermittel, das den pH der Formulierung bei einem pH von 5,5 hält.

7. Zusammensetzung nach einem beliebigen der vorangehenden Ansprüche, wobei die Zusammensetzung weiterhin ein pharmazeutisch verträgliches Lösungsmittel umfasst.

8. Zusammensetzung nach einem beliebigen der vorangehenden Ansprüche, wobei das physiologisch verträgliche Puffermittel aus Citrat-, Acetat-, Phosphat-, TRIS-(Tromethamin), Arginin- und Carbonatpuffem ausgewählt ist.

9. Zusammensetzung nach Anspruch 8, wobei das physiologisch verträgliche Puffermittel ein nicht flüchtiges Puffermittel ist.

10. Zusammensetzung nach einem beliebigen der vorangehenden Ansprüche, weiterhin umfassend mindestens einen Tonizitätsmodifizierer, bevorzugt wobei der eine Tonizitätsmodifizierer aus Natriumchlorid, Saccharose und einem Polyolhilfsstoff ausgewählt ist.

11. Zusammensetzung nach Anspruch 10, wobei der Polyolhilfsstoff aus Sorbitol, Glycerin und Mannitol ausgewählt ist.

12. Zusammensetzung nach einem beliebigen der Ansprüche 10 bis 11, wobei der mindestens eine Tonizitätsmodifizierer mit dem nicht flüchtigen Puffermittel colyophilisiert.

13. Zusammensetzung nach einem beliebigen der vorangehenden Ansprüche, weiterhin umfassend mindestens ein pharmazeutisch verträgliches Konservierungsmittel, bevorzugt wobei das mindestens eine pharmazeutisch verträgliche Konservierungsmittel aus Kresol, Benzylalkohol und EDTA ausgewählt ist.

14. Zusammensetzung nach einem beliebigen der vorangehenden Ansprüche, weiterhin umfassend mindestens ein pharmazeutisch verträgliches Stabilisierungsmittel, bevorzugt wobei das mindestens eine pharmazeutisch verträgliche Stabilisierungsmittel aus Xylit, Lysin, Histidin und mikrokristalliner Zellulose ausgewählt ist.

15. Zusammensetzung nach einem beliebigen der vorangehenden Ansprüche, wobei die Zusammensetzung zur Verabreichung durch Injektion geeignet ist.

16. Zusammensetzung nach einem beliebigen der vorangehenden Ansprüche, wobei die Zusammensetzung lyophilisiert ist.

17. Verfahren zum Herstellen einer pharmazeutischen Zusammensetzung, umfassend die Schritte des Wiederherstellens der Zusammensetzung gemäß Anspruch 16 in eine Lösung mit einem pharmazeutisch verträglichen Vehikel.

18. Zusammensetzung gemäß einem beliebigen der Ansprüche 1 bis 16 zur Verwendung als Medikament.

19. Zusammensetzung gemäß einem beliebigen der Ansprüche 1 bis 16 zur Verwendung als Medikament zur Behandlung oder zur Vorbeugung von Osteoporose.

## Revendications

1. Composition pharmaceutique comprenant, lorsqu'elle est dissoute dans un véhicule approprié, de la PTH à une concentration de 1 mg/ml à 10 mg/ml, de la méthionine fournie à une concentration de 1 mg/ml à 10 mg/ml, au moins un tensioactif poloxamère fourni à une concentration de 0,02 mg/ml à 1 mg/ml, et un agent tampon physiologiquement acceptable qui maintient le pH de la formulation à un pH de 5 à 6,5.

2. Composition selon la revendication 1, dans laquelle la composition est sous la forme d'une formulation de type solution stable.

3. Composition selon la revendication 1 ou 2, dans laquelle le au moins un tensioactif poloxamère est choisi parmi le Poloxamère 188, le Poloxamère 407, le Poloxamère 237, le Poloxamère 338 et le Poloxamère 124.

4. Composition selon l'une quelconque des revendications précédentes, dans laquelle la PTH est choisie parmi la hPTH(1-84) et une molécule de PTH de mammifère, non-humaine, ainsi que les variants et les homologues biologiquement actifs de la hPTH(1-84) et des molécules de PTH de mammifère, non-humaines.

5. Composition selon la revendication 4, dans laquelle la composition comprend un homologue biologiquement actif de la hPTH(1-84) partageant une identité de séquence avec la hPTH(1-84) d'au moins 85 %, de préférence dans laquelle l'homologue biologiquement actif partage une identité de séquence avec la hPTH(1-84) d'au moins 95 %, de préférence dans laquelle l'homologue biologiquement actif partage une identité de séquence avec la hPTH(1-84) d'au moins 98 %.

6. Composition selon l'une quelconque des revendications 1 à 5, comprenant de la PTH à une concentration de 1,4 mg/ml, de la méthionine à une concentration de 2 mg/ml, un poloxamère à une concentration de 0,3 mg/ml et un agent tampon physiologiquement acceptable qui maintient le pH de la formulation à un pH de 5,5.

7. Composition selon l'une quelconque des revendications précédentes, dans laquelle ladite composition comprend en outre un solvant pharmaceutiquement acceptable.

8. Composition selon l'une quelconque des revendications précédentes, dans laquelle l'agent tampon physiologiquement acceptable est choisi parmi les tampons citrate, acétate, phosphate, TRIS (trométhamine), arginine et carbonate.

9. Composition selon la revendication 8, dans laquelle l'agent tampon physiologiquement approprié est un agent tampon non volatile.

10. Composition selon l'une quelconque des revendications précédentes, comprenant en outre au moins un modificateur de tonicité, de préférence dans laquelle le au moins un modificateur de tonicité est choisi parmi le chlorure de sodium, le saccharose et un excipient polyol.

11. Composition selon la revendication 10, dans laquelle l'excipient polyol est choisi parmi le sorbitol, le glycérol et le mannitol.

12. Composition selon l'une quelconque des revendications 10 à 11, dans laquelle au moins un modificateur de tonicité se co-lyophilise avec l'agent tampon non volatile.

13. Composition selon l'une quelconque des revendications précédentes, comprenant en outre au moins un agent conservateur pharmaceutiquement acceptable, de préférence dans laquelle le au moins un conservateur pharmaceutiquement acceptable est choisi parmi le crésol, l'alcool benzylique et l'EDTA.

14. Composition selon l'une quelconque des revendications précédentes, comprenant en outre au moins un stabilisant pharmaceutiquement acceptable, de préférence dans laquelle le au moins un stabilisant pharmaceutiquement acceptable est choisi parmi le xylitol, la lysine, l'histidine et la cellulose microcristalline.

15. Composition selon l'une quelconque des revendications précédentes, dans laquelle ladite composition est adaptée pour une administration par injection.

16. Composition selon l'une quelconque des revendications précédentes, dans laquelle ladite composition est lyophilisée.

17. Procédé de préparation d'une composition pharmaceutique, comprenant les étapes de reconstitution de la composition selon la revendication 16 en une solution avec un véhicule pharmaceutiquement acceptable.

18. Composition selon l'une quelconque des revendications 1 à 16 pour une utilisation comme médicament.

19. Composition selon l'une quelconque des revendications 1 à 16 pour une utilisation comme médicament pour le traitement ou la prévention de l'ostéoporose
